# EUROPÄISCHE PATENTSCHRIFT

(11) **EP 3 164 097 B1**
(45) Veröffentlichungstag und Bekanntmachung des Hinweises auf die Patenterteilung: **11.05.2022**
(21) Anmeldenummer: 15735892.0
(22) Anmeldetag: 30.06.2015
(51) Int. Cl.: A61C 13/00, A61K 6/16, A61K 6/17, A61K 6/887

(54) **FRÄSROHLINGE BASIEREND AUF EINEM AUSPOLYMERISIERTEN, BRUCHZÄHEN PROTHESENMATERIAL**
MILL BLANKS BASED ON A POLYMERIZED, FRACTURE-TOUGH PROSTHESIS MATERIAL
ÉBAUCHES À FRAISER À BASE D'UN MATÉRIAU DE PROTHÈSE POLYMÉRISÉ TENACE À LA RUPTURE

(30) Priorität: 01.07.2014 DE 102014109233
(43) Veröffentlichungstag der Anmeldung: 10.05.2017
(73) Patentinhaber: Kulzer GmbH, 63450 Hanau (DE)
(72) Erfinder: RUPPERT, Klaus, 63477 Maintal (DE); HOHMANN, Alfred, 61389 Schmitten (DE); DEKERT, Stephan, 61273 Wehrheim (DE)
(74) Vertreter: Bendele, Tanja
(86) Internationale Anmeldenummer: PCT/EP2015/064875
(87) Internationale Veröffentlichungsnummer: WO 2016/001242

(56) Entgegenhaltungen:
- EP-A2- 1 702 633
- DE-A1-102012 013 514
- DE-A1-102012 022 693

## Beschreibung

Gegenstand der Erfindung ist ein dreidimensionaler Formkörper, insbesondere ein Fräsrohling, aus einem polymeren Prothesenmaterial, der geeignet ist für eine abtragende Bearbeitung mittels CNC und/oder CAD/CAM, mit einer Bruchzähigkeit von grösser gleich 1,9 MPa ·m^{1/2} und einer Gesamtbrucharbeit von grösser gleich 900 J/m² aufweist. Der Fräsrohling umfasst vorzugsweise durch eine elastische Phase modifizierte Kern-Schale-Partikel und basiert optional auf einem Gehalt an einpolymerisiertem Urethan(meth)acrylat. Ferner ist Gegenstand der Erfindung die Verwendung der Fräsrohlinge zur Herstellung von zumindest Teilen von dentalen prothetischen Teilen, wie einer Prothese, Aufbissschienen, Bohrschablonen, künstlichen Zähnen, Kronen und Brücken, oder auch von kiefer-orthopädischen Apparaten und Instrumenten im medizinischen Bereich. Besonders bevorzugt ist der Fräsrohling geeignet für eine Bearbeitung mittels Fräsen.

Prothesen fallen aufgrund der eingeschränkten Motorik der in der Regel älteren Prothesenträger beim Reinigen zuweilen auf einen harten Untergrund (Fliesen, Waschbecken), so dass es hier zu Abplatzern oder Brüchen kommen kann. Diese Abplatzer führen zu einer Zusatzarbeit und Zusatzkosten für das Dentallabor und sind daher unerwünscht. Ferner kann es insbesondere bei Implantat-getragenem Zahnersatz aufgrund der deutlich höheren Kaukräfte zu Beschädigungen der Prothese kommen.

Aus diesen Gründen besteht die Anforderung nach einem Prothesenwerkstoff, welcher kurzzeitige Belastungen, wie die vorgenannten kurzzeitigen, hohen mechanischen Belastungen, ohne Materialschädigung toleriert. Diese Prothesenwerkstoffe werden als sogenannte High Impact-Werkstoffe bezeichnet. Die Anforderungen an diese Werkstoffe sind in der DIN ISO 20795-1 beschrieben. Produkte, welche diese Anforderungen erfüllen, sind schon seit einigen Jahren auf dem Markt, diese sind allerdings überwiegend der Gruppe der heisshärtenden Prothesenwerkstoffe zuzuordnen. Ferner besteht ein Bedarf an Fräsblöcken für eine Bearbeitung mittels CAD/CAM. So besteht für bestimmte prothetische Anwendungen, wie Implantat-getragene Prothesen, Aufbissschienen oder KFO-Apparaturen (kiefer-orthopädische Apparaturen) ein Bedarf an Materialien, die ebenfalls kurzzeitige, hohe mechanische Belastungen ohne eine Materialschädigung verkraften. Bislang sind jedoch nur PMMA-Fräsblöcke für die CAD/CAM-Bearbeitung bekannt. Diese Materialien sind jedoch nicht immer den Kaubelastungen der vorgenannten prothetischen Teile oder kieferorthopädischen Apparate gewachsen.

DE102012022693A1 offenbart ein Zweikomponentensystem umfassend eine feste, schlagzäh modifizierte Komponente A und eine, Bestandteile zur Schlagzähmodifizierung enthaltende, flüssige Komponente B. DE102012013514A1 offenbart ein polymeres Prothesenmaterial, das eine Bruchzähigkeit von ≥ 1,9 MPa·m^{1/2} und eine Brucharbeit von ≥ 900 J/m² aufweist. EP1702633A2 offenbart ein autopolymerisierendes 2-Komponenten-Prothesenbasismaterial bestehend aus A) einer flüssigen Monomerkomponente und B) einer pulverförmigen füllstoffhaltigen Komponente, die mindestens ein durch eine elastische Phase modifiziertes Perlpolymerisat enthält und ausgehärtet eine Bruchzähigkeit von ≥ 2 MPa·m^{1/2} und eine Brucharbeit von ≥ 900 J/m² aufweist.

Aufgabe der Erfindung war die Bereitstellung eines fräsbaren Werkstoffes, vorzugsweise in Form eines Fräsrohlings, wie eines im medizinischen Bereich geeigneten Werkstoffes, der die Vorgaben der Norm DIN ISO 20795-1 hinsichtlich Bruchzähigkeit übertrifft. Ferner bestand die Aufgabe, dass der fräsbare Werkstoff, insbesondere in Form eines Fräsrohlings, vorliegt. Ferner bestand die Aufgabe, dass der Fräsrohling die ästhetischen Anforderungen an die Transparenz prothetischer Materialien erfüllt und keine oder nur eine sehr geringe Anzahl an Blasen enthält. Eine weitere Aufgabe bestand darin, einen hoch-bruchzähen Werkstoff in Form eines Fräsrohlings bereitzustellen, insbesondere einen Fräsrohling aus einem Prothesenwerkstoff, dessen Transparenz im Vergleich zum Grundwerkstoff nicht beeinträchtigt wird. Eine weitere Aufgabe bestand darin, den Fräsrohling ggf. auch mehrfarbig, d.h. fleischfarben und zahnfarben bereitzustellen.

Es wurde überraschend gefunden, dass ein Fräsrohling aus einem Werkstoff, insbesondere einem Prothesenmaterial, mit den geforderten Eigenschaften bezüglich der Bruchzähigkeit und vorzugsweise bezüglich der Vorgaben zur Transparenz, bereitgestellt werden kann. Vorzugsweise basiert der Fräsrohling auf einem auspolymerisierten Prothesenmaterial umfassend Kern-Schale-Partikel und optional einpolymerisiertem Urethan(meth)acrylat.

Ferner werden an fräsbare Materialien erhöhte Anforderungen an die Eigenschaften der Oberfläche und der nach dem Fräsvorgang erhaltenen Oberflächen gestellt. So dürfen sich keine Mikrorisse durch die Bearbeitung ausbilden.

Möglicherweise zur Erhöhung der Bruchzähigkeit geeignete Flüssigadditive , wie z.B. AcrylnitrilButadien-Copolymere, neigten jedoch zum Vergilben des Prothesenmaterials. Dies geschieht typischerweise in Gegenwart von oxidierenden Substanzen wie z.B. Peroxiden oder Luftsauerstoff. Zudem weisen die Flüssigadditive, wie z.B. Silikonacrylate eine deutlich von PMMA unterschiedliche Brechzahl auf und führen dadurch typischerweise zur Ausbildung eines opaken Werkstoffs. Die Urethanacrylat-basierten Flüssigadditive, wie z.B. Urethanacrylate, führen zwar oft zu einer Verbesserung der Bruchzähigkeit von Materialien, wobei die Mindestanforderungen der ISO 20795-1 bezüglich der Bruchzähigkeit jedoch nicht erreicht werden können. Ein weiterer Nachteil einiger Additive zeigt sich in einer Wasseraufnahme während des Polymerisationsprozesses, dies führt zu einer unerwünschten Weissverfärbung des Prothesenwerkstoffes während der Tragezeit.

Die Aufgaben wurden gelöst durch einen Fräsrohling nach Anspruch 1, ein Verfahren zur Herstellung der Fräsrohlinge nach Anspruch 6 und die Verwendung der Fräsrohlinge nach Anspruch 11 sowie durch ein nach dem Verfahren erhältliches auspolymerisiertes Prothesenmaterial nach Anspruch 9, wobei konkrete Ausführungsformen in den Unteransprüchen und in der Beschreibung erläutert werden.

Gegenstand der Erfindung ist somit ein Fräsrohling aus polymerem Prothesenmaterial basierend auf der Umsetzung mindestens einer (A) flüssigen Monomerkomponente und einer (B) pulverförmigen Komponente, wobei die (A) flüssige Monomerkomponente größer 85 Gew.-% mindestens eine monofunktionelle (Meth)acrylat-funktionelle Monomerkomponente, 0,001 bis 20 Gew.-% Urethan(meth)acrylat, und 0,001 bis 10 Gew.-% durch eine elastische Phase modifizierte Kern-Schale-Partikel mit elastischer Phase als Kern in harter Aussenschale, wobei die Kern-Schale-Partikel Primärpartikel mit einer mittleren Partikelgröße von kleiner gleich d₅₀ ≤ 500 nm bis 10 nm aufweisen, umfasst, wobei die Gesamtzusammensetzung der flüssigen Monomerkomponente (A) 100 Gew.-% beträgt, vorzugsweise bestehend aus polymerem Prothesenmaterial, insbesondere ein auspolymerisiertes Prothesenmaterial, wobei das Prothesenmaterial in Form eines dreidimensionalen Formkörpers geeignet als CAD/CAM-Fräsrohling vorliegt und mindestens ein Polymer umfassend mindestens ein einpolymerisiertes Urethan(meth)acrylat enthält und 0,001 bis 10 Gew.-% der durch eine elastische Phase modifizierten Kern-Schale-Partikel in Bezug auf die Gesamtzusammensetzung umfasst, wobei der Formkörper eine Bruchzähigkeit von grösser gleich 1,9 MPa m^{1/2} und einer Gesamtbrucharbeit von grösser gleich 900 J/m², insbesondere weist der Fräsrohling eine Bruchzähigkeit von grösser gleich 2,3 MPa · m^{1/2} und optional eine Gesamtbrucharbeit von grösser gleich 1000 J/m² auf.

Der erfindungsgemäße Fräsrohling kann vorzugsweise mehrfarbig ausgebildet sein, insbesondere sind definierte Bereiche des Fräsrohlings Zahnfarben und/oder definierte Bereiche farblich der Gingiva nachgebildet, insbesondere sind diese Bereiche fleischfarben. Entsprechend einer weiteren Alternative kann der Fräsrohling rein Zahnfarben oder rein farblich der Gingiva nachgebildet sein. Dabei kann sowohl die Zahn- als auch die Fleischfarbe natürlichen Farbverläufen angepasst sein. Ferner umfassen die erfindungsgemässen Fräsrohlinge (i) durch eine elastische Phase modifizierte Kern-Schale Partikel und (ii) mindestens ein Polymer umfassend mindestens ein einpolymerisiertes Urethan(meth)acrylat.

Die Kern-Schale-Partikel umfassen Primärpartikel mit einer mittleren Partikelgrösse von kleiner gleich d₅₀ ≤ 500 nm bis 10 nm, vorzugsweise von 100 bis 450 nm, besonders bevorzugt von 200 bis 400 nm. Die Primärpartikel der Kern-Schale-Partikel können als Aggregate von Primärpartikeln vorliegen. Diese Aggregate weisen vorzugsweise eine mittlere Partikelgrösse von kleiner gleich d₅₀ 400 µm (Micrometer) auf.

Zur Einstellung der Eigenschaften des Fräsrohlings umfasst der Fräsrohling zu (i) 0,001 bis 10 Gew.-% durch mindestens eine elastische Phase modifizierte Kern-Schale-Partikel in Bezug auf die Gesamtzusammensetzung besonders bevorzugt bis zu 5 Gew.-%.

Weiter ist es bevorzugt, wenn der Fräsrohling bis zu iii) 0,001 bis 20 Gew.-% mindestens ein einpolymerisiertes Urethan(meth)acrylat, insbesondere ein Urethandimethacrylat, in Bezug auf die Gesamtzusammensetzung aufweist, vorzugsweise bis zu 10 Gew.-%, besonders bevorzugt bis zu 5 Gew.-%. Vorzugsweise ist das Urethandimethacrylat in das Prothesenmaterial einpolymerisiert.

Erfindungsgemäss wird die Aufgabe durch die synergistische Verwendung von Kern-Schale-Partikeln und mindestens einem Urethanmethacrylat im Prothesenbasismaterial gelöst. Besonders gute Ergebnisse im auspolymerisierten Prothesenmaterial können durch eine Kombination von Kern-Schale-Partikeln in der flüssigen Monomerkomponente in Kombination mit mindestens einem Urethanacrylat oder Urethanmethacrylat in der Flüssigkeit erzielt werden. Eine hohe Transparenz des Prothesenwerkstoffs konnte durch Auswahl spezifischer Kern-Schale-Partikel mit einer Brechzahl ähnlich der des auspolymerisierten Prothesenwerkstoffes gewährleistet werden. Daher weisen die Kern-Schal-Partikel vorzugsweise eine Brechzahl von etwa 1,49 auf (R.I. ~ 1,4900).

Erfindungsgemäss besonders bevorzugte Kern-Schale-Partikel liegen aggregiert vor. Die Aufgaben können durch die Verwendung von aggregierten Kern-Schale-Partikeln (unregelmässig geformte Aggregate, d₅₀ ~ 50 - 400 µm, insbesondere 50 - 300 µm) gelöst werden, die Primärteilchengrösse beträgt ca. 200 - 400 nm. Die Kern-Schale-Partikel liegen vermutlich aufgrund von Oberflächenwechselwirkungen im Feststoff aggregiert vor. Das Additiv wird mit der Flüssigkeit gemischt und bildet eine stabile Suspension, welche innerhalb von wenigen Wochen nur schwach sedimentiert. Durch die Suspendierung in MMA zerfallen die Aggregate relativ rasch in die Primärpartikel, die dann homogen verteilbar sind und vorzugsweise homogen verteilt in den Fräsrohlingen vorliegen.

Durch die erfindungsgemässe Verwendung der Kern-Schale-Partikel als High-Impact Additiv in Kombination mit mindestens einem Urethanacrylat, vorzugsweise einem Urethanmethacrylat, lassen sich Prothesenwerkstoffe herstellen, welche die Anforderungen der ISO 20795-1 hinsichtlich High-Impact Eigenschaften erfüllen. Darüber hinaus kann ein Prothesenwerkstoff bereitgestellt werden, dessen Biegefestigkeit und E-Modul in der gleichen Grössenordnung wie Nicht-High-Impact Werkstoffe liegen und der gleichzeitig hochtransparent und farbstabil ist. Die erfindungsgemässen Prothesen zeigen keine Weissverfärbungen durch Kontakt mit wasserhaltigen Materialien wie z.B. Dubliergele oder Gipse während und nach dem Polymerisationsprozess.

Zur Abgrenzung von Prothesenmaterialien, wie hier als Material des Fräsrohlings verwendet, zu üblichen dentalen Materialien wird hervorgehoben, dass Prothesenmaterialien wesentliche Mengen an polymeren pulverförmigen Komponenten umfassen, wie PMMA (Poly(methyl)-methacrylat) und/oder Poly(ethyl)methacrylat, insbesondere zu grösser gleich 50 Gew.-% in der Gesamtzusammensetzung. Dentale Materialien zur Herstellung von Füllungen basieren im Wesentlichen auf polymerisierbaren Monomeren, die vorzugsweise mit einem Anteil von kleiner 35 Gew.-% in den polymerisierbaren Zusammensetzungen vorliegen.

Übliche Prothesenmaterialien werden in der Regel in einem Kit mit einer pulverförmigen Komponente und einer flüssigen Komponente angeboten. Erfindungsgemäß wird ein Fräsrohling in Form eines auspolymerisierten Prothesenmaterials, optional farbig an die Zahnsituation und/oder Gingiva angepasst, bereitgestellt.

Erfindungsgemäss besonders gut verwendbar sind Kern-Schale-Partikel, welche ggf. als Aggregate vorliegen, wobei diese Aggregate jedoch innerhalb der kurzen Anquellzeit in die einzelnen Partikel auftrennbar sind, insbesondere zerfallen sie nach Zugabe der flüssigen Monomerkomponente in die einzelnen Partikel.

Entsprechend einer weiteren Ausführungsform können die erfindungsgemässen Fräsrohlinge zusätzlich einen oder mehrere Stoff(e) aus den Gruppen der Füllstoffe, Pigmente, Stabilisatoren, Regler, antimikrobiellen Additive, UV-Absorber, Thixotropiermittel, Katalysatoren und Vernetzer enthalten.

Erfindungsgemässe Fräsrohlinge umfassen vorzugsweise zu grösser gleich 55 Gew.-% PMMA in Bezug auf die Gesamtzusammensetzung, insbesondere grösser gleich 60, 70, 75, 80, 85, 90, 95, 97 Gew.-% PMMA in Bezug auf die Gesamtzusammensetzung und optional 0,001 bis 5 Gew.-% Kern-Schale-Partikel, bevorzugt 0,5 bis 2 Gew.-%, besonders bevorzugt 0,5 bis 1,5 Gew-%. Des Weiteren kann es bevorzugt sein, wenn die Fräsrohlinge zudem 0,001 bis 10 Gew.-% Urethan(meth)acrylat enthalten, bevorzugt 0,5 bis 5 Gew.-5, besonders bevorzugt 0,5 bis 2 Gew.-%.

Entsprechend einer weiteren besonders bevorzugten Ausführungsform basieren die Fräsrohlinge auf einem auspolymerisierten, bruchzähen Prothesenmaterial, das vorzugsweise nach dem erfindungsgemässen Verfahren erhältlich ist. Nach einer Alternative wird ferner offenbart ein Verfahren zur Herstellung eines auspolymerisierten Prothesenbasismaterials sowie ein Prothesenmaterial, insbesondere ein Prothesenmaterial in Form eines Fräsrohlings, indem die Komponenten A) mindestens eine flüssige Monomerkomponente, und B) mindestens eine pulverförmige Komponente, des Prothesenmaterials, gemischt werden und nachfolgend auspolymerisiert werden.

Gegenstand der Erfindung ist ebenfalls ein Verfahren zur Herstellung eines auspolymerisierten Prothesenmaterials in Form eines dreidimensionalen Formkörpers, vorzugsweise in Form eines Fräsrohlings, indem ein polymerisierbares Prothesenmaterial umfassend die Komponente (A) und/oder (B), wobei die Komponente A) mindestens eine flüssige Monomerkomponente, und B) mindestens eine pulverförmige Komponente ist, und das polymerisierbare Prothesenmaterial in Komponente (A) größer 85 Gew.-% mindestens eine monofunktionelle (Meth)acrylat-funktionelle Monomerkomponente, 0,001 bis 20 Gew.-% Urethan(meth)acrylat, und durch eine elastische Phase modifizierte Kern-Schale-Partikel mit elastischer Phase als Kern in harter Aussenschale, wobei die Kern-Schale-Partikel Primärpartikel mit einer mittleren Partikelgröße von kleiner gleich d₅₀ ≤ 500 nm bis 10 nm aufweisen umfasst, wobei die Gesamtzusammensetzung der flüssigen Monomerkomponente (A) 100 Gew.-% beträgt, und die Komponente (A) und (B) (a) gemischt werden, (b) in eine dreidimensionale Form, insbesondere in die Form einer Halbfertigproduktform von Fräsrohlingen oder in die Form von Fräsrohlingen, überführt werden, (c) und nachfolgend das polymerisierbare Prothesenmaterial auspolymerisiert wird.

Das Verfahren kann vorzugsweise derart durchgeführt werden, dass das Gemisch umfassend die A) Monomerkomponente und die B) pulverförmige Komponente, insbesondere als auspolymerisierbares Prothesenbasismaterial in eine Negativform, wie eine Gussform, insbesondere eine Halbfertigproduktform von Fräsrohlingen oder eine Negativform eines Fräsrohlings oder mindestens eines dentalen, prothetischen Formkörpers eingeführt wird und, insbesondere unter erhöhtem Druck, insbesondere grösser gleich 2 bar, wie 2, 5 bis 10 bar, vorzugsweise 2 bis 4 bar, auspolymerisiert wird.

Als eine dreidimensionale Form gelten nach der Erfindung vorzugsweise Halbfertigproduktformen von Fräsrohlingen, wie stangenförmige, scheibenförmige, zylinderförmige oder weitere dem Fachmann bekannte Halbfertigproduktformen und Fertigproduktformen von Fräsrohlingen, besonders bevorzugt sind scheibenförmige, vorzugsweise zylinderförmige Fräsrohlinge. Unter zylinderförmig können mathematische oder auch kreisförmige Zylinderformen erfasst werden, wie kreisförmige Scheiben. Ebenso können die Fräsrohlinge quaderförmig oder sonst eine polyedrische Form bzw. jede beliebige dreidimensionale geeignete Form aufweisen.

Dabei ist es erfindungsgemäss bevorzugt, wenn in dem Verfahren die A) Monomerkomponente und die B) pulverförmige Komponente im Gewichtsverhältnis von 1 : 50 bis 50 : 1, insbesondere im Gewichtsverhältnis 8 bis 11 pulverförmige Komponente zu 5 bis 8 Monomerkomponente gemischt werden.

Als Komponente A), die flüssige Monomerkomponente, kommen vorzugsweise in Betracht mindestens ein Monomer oder eine Mischung von Monomeren umfasst von a) Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornyl-acrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, Benzyl-, Furfuryl- oder Phenyl(meth)acrylat eine Mischung enthaltend mindestens eines dieser (Meth-)acrylate und/oder Co-Polymere umfassend eines oder mindestens zwei der vorgenannten Monomere, und/oder b) zwei- und/oder Mehrfachvernetzer umfassend 1,4-Butandiol-dimethacrylat (1,4-BDMA) oder Pentaerythritol-tetraacrylat, Bis-GMA-Monomer (Bisphenyl-A-Glycidyl-Methacrylat), Triethylenglycoldimethacrylat (TEGDMA) und Diethylenglycoldimethacrylat (DEGMA), Tetraethylenglykoldi(meth)acrylat, Decandioldi(meth)acrylat, Dodecandioldi(meth)acrylat, Hexyldecandioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate eine Mischung enthaltend mindestens eines dieser (Meth-)acrylate und/oder Co-Polymere umfassend eines oder mindestens zwei der vorgenannten Monomere.

Als Komponente B), die pulverförmige Komponente, kommen vorzugsweise in Betracht polymere Partikel umfassend Polymere in Form von Polymerpulver umfassend Polyalkyl(meth)acrylate, die optional vernetzt sind und als Homo- oder Co-Polymere vorliegen, wobei die Polymere auf mindestens einem der Monomere, umfassend eine (Meth-)acrylat-Gruppe, basieren, ausgewählt aus Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylenglykol-mono-acrylat, Hydroxyethylacrylat, Hydroxypropylacrylat, Hydroxyethylmethacrylat, Hydroxypropylmethacrylat, eine Mischung enthaltend mindestens eines dieser (Meth-)acrylate und/oder Co-Polymere umfassend eines oder mindestens zwei der vorgenannten Monomere, Polyamidpartikel, Polyamidfasern, besonders bevorzugt ist Polymethylmethacrylat (PMMA). Darüber hinaus können die polymeren Partikel auch Gemische dentaler Monomere wie z.B. MMA und zusätzlich mindestens einen Vernetzer umfassen.

Besonders bevorzugt sind pulverförmige Komponenten B) umfassend Polymethylmethacrylat (PMMA) Perlen als polymere Partikel und/oder Splitterpolymerisate sowie Co-Polymere umfassend als einpolymerisierte Comonomere Styrol, alpha-Methylstyrol, Vinyltoluol, substituierte Vinyltoluole wie Vinylbenzylchloride, Vinylhalogenide wie Vinylchlorid, Vinylester, wie Vinylacetat, heterocyclische Vinylverbindungen wie 2-Vinylpyridin, Vinylacetat und Vinylpropionat, Butadien, Isobutylen, 2-Chlorbutadien, 2-Methylbutadien, Vinylpyridin, Cyclopenten, (Meth)acrylsäureester, wie Methylmethacrylat, Butylmethacrylat, Butylacrylat und Hydroxyethylmethacrylat, ferner Acrylnitril, Maleinsäure und Maleinsäurederivate, wie beispielsweise Maleinsäureanhydrid, Fumarsäure und Fumarsäurederivate, wie Fumarsäureester, Acrylsäure, Methacrylsäure, Aryl(meth)acrylate, wie Benzylmethacrylat oder Phenylmethacrylat sowie optional Mischungen dieser Comonomere.

Ferner umfasst das Prothesenmaterial vorzugsweise in Komponente (A) und/oder (B) (iii) mindestens einen Initiator oder ein Initiatorsystem für die Autopolymerisation, die Strahlenhärtung, insbesondere UV-Härtung, Heisspolymerisation oder Dualhärtung.

Ein weiterer besonders bevorzugter Gegenstand der Erfindung ist ein auspolymerisiertes Prothesenmaterial erhältlich nach einem Verfahren nach einem erfindungsgemässen Verfahren, das in Form eines dreidimensionalen Formkörpers vorliegt, insbesondere in Form eines Fräsrohlings oder einer Halbfertigproduktform.

Dabei ist es weiter bevorzugt, wenn das auspolymerisierte Prothesenmaterial eine Bruchzähigkeit von ≥ 1,9 MPa · m^{1/2} und eine Gesamtbrucharbeit von ≥ 900 J/m² aufweist.

Der erfindungsgemässe Fräsrohling aus dem Prothesenmaterial weist als auspolymerisiertes Prothesenmaterial vorzugsweise eine Bruchzähigkeit (kₘₐₓ; Höchstfaktor der Beanspruchungsintensität) von grösser gleich 1,9 MPa · m^{1/2} auf, insbesondere grösser gleich 2 MPa · m^{1/2}, und vorzugsweise zugleich eine Gesamtbrucharbeit (W_{f}) von grösser gleich 900 J/m². Besonders bevorzugt liegt die Bruchzähigkeit bei grösser gleich (≥) 2,1 MPa · m^{1/2}, vorzugsweise bei ≥ 2,3 MPa · m^{1/2}, ≥ 2,4 MPa · m^{1/2}. Weiter ist es bevorzugt, wenn die Gesamtbrucharbeit zugleich grösser ≥ 900 J/m² ist, insbesondere grösser gleich ≥ 950 J/m², ≥ 1000 J/m², besonders bevorzugt grösser gleich 1030 J/m². Besonders bevorzugt ist zudem die Biegefestigkeit zudem grösser 65 MPa, besonders bevorzugt grösser 70 MPa, weiter bevorzugt grösser gleich 75 MPa. Ein besonders bevorzugtes Prothesenmaterial weist eine Bruchzähigkeit von > 2,3 MPa*m^{1/2} und eine Gesamtbrucharbeit von > 1000 J/m² auf.

Weiter ist es bevorzugt, wenn die Transparenz der unpigmentierten, auspolymerisierten Fräsrohlinge, und der daraus hergestellten prothetischen Teile oder kieferorthopädischen Apparate im Bereich von grösser gleich 85%, insbesondere grösser gleich 90% liegt (gemessen an 3 mm dicken Platte).

Das Prothesenmaterial ist nach einer Ausführungsform ein autopolymerisierbares bzw. kaltpolymerisierbares oder ein heisspolymerisierbares 2-Komponenten-Prothesenbasismaterial, insbesondere ein polymerisierbares Prothesenmaterial, umfassend A) mindestens eine flüssige Monomerkomponente, B) mindestens eine pulverförmige Komponente, insbesondere zur Verwendung zur Herstellung von Fräsrohlingen, ,
wobei das Prothesenmaterial in Komponente (A) und/oder (B)
(i) mindestens einen Initiator oder ein Initiatorsystem für die Autopolymerisation bzw. Kaltpolymerisation oder eine Heisspolymerisation,
(ii) durch eine elastische Phase modifizierte Kern-Schale Partikel und
(iii) mindestens ein Urethan(meth)acrylat enthält, insbesondere ein Urethandimethacrylat, bevorzugt ein Bis(methacryloxy-2-ethoxycarbonylamino)-alkylen, Diurethanacrylat Oligomer, Alkyl-funktionelle Urethandimethacrylat Oligomere, Aromatisch-funktionalisierte Urethandimethacrylat Oligomere, aliphatische ungesättigte Urethanacrylate, Bis(methacryloxy-2-ethoxycarbonylamino) substituierter Polyether, aromatische Urethandiacrylat Oligomere, aliphatische Urethandiacrylat Oligomere, monofunktionelle Urethanacrylate, aliphatische Urethandiacrylate, hexafunktionelle aliphatische Urethanharze, aliphatisches Urethantriacrylat, UDMA, aliphatisches Urethanacrylat Oligomer, ungesättigte aliphatische Urethanacrylat.

Komponenten des Initiatorsystems können, wie nachfolgend erläutert, auf die Komponenten A) und B) aufgeteilt werden. Ebenso Gegenstand der Erfindung ist ein Verfahren zur Herstellung des Fräsrohlings aus dem vorgenannten polymerisierbaren Prothesenmaterial sowie ein dreidimensionaler Rohling erhältlich durch Mischen, Formen und Auspolymerisieren des polymerisierbaren Prothesenmaterials.

Besonders bevorzugt umfasst die Komponente (A) mindestens eine flüssige Monomerkomponente, (i) mindestens einen Initiator oder ein Initiatorsystem für die Autopolymerisation oder Heisspolymerisation oder einen Teil der Komponenten des Initiatorsystems, (ii) durch mindestens eine elastische Phase modifizierte Kern-Schale-Partikel und (iii) mindestens ein Urethan(meth)acrylat, insbesondere Urethandimethacrylat.

Dabei ist es erfindungsgemäß vorgesehen, dass die Komponente (ii) durch mindestens eine elastische Phase modifizierte Kern-Schale-Partikel zu 0,001 bis 10 Gew.-%, bevorzugt bis 5 Gew.-%, in Bezug auf die Gesamtzusammensetzung der Komponente (A) und (iii) mindestens ein Urethan(meth)acrylat, vorzugsweise ein Urethandimethacrylat, von 0,001 bis 20 Gew.-%, vorzugsweise bis 10 Gew.-%, weiter bevorzugt bis 5 Gew.-%, in Bezug auf die Gesamtzusammensetzung der Komponente (A) vorliegt (d.h. auf 100 Gew.-% der Komponente (A)).

Der erfindungsgemässe Fräsrohling aus Prothesenbasismaterial, insbesondere aus autopolymerisiertem Prothesenbasismaterial, zeigt trotz des zugesetzten High Impact - Modifizierers (Kern-Schale Partikel und Urethan(meth)acrylat) keine negative Beeinflussung des Suntests nach ISO 20795-1. Die Kern-Schale-Partikel werden auch als High-Impact Modifizierer bezeichnet.

Erfindungsgemäße Fräsrohlinge aus Prothesenmaterialien weisen Kern-Schale-Partikel auf, in denen die Verteilung der elastischen Phase der modifizierten Kern-Schale-Partikel ausgewählt ist aus: elastischer Phase als Kern (z.B. aus Butylacrylat) in harter Aussenschale (z.B. aus PMMA) (Kern-Schale-Teilchen).

Erfindungsgemässe Kern-Schale-Partikel können ebenso den nachfolgenden multi-layer Aufbau aufweisen, e) einen innen liegenden Kern mit mehreren Schichten als Schalen und einer Aussenschale, wobei insbesondere (i) mindestens eine der Schalen, vorzugsweise die Aussenschale hart ist und die verbleibenden Schalen und der Kern jeweils unabhängig aus elastischen Phasen bestehen. In Alternativen können die elastischen Phasen und die harten Phasen auf die Schalen und den Kern anderweitig aufgeteilt sein.

Ferner weisen bevorzugte Kern-Schale-Partikel eine Brechzahl ähnlich der des auspolymerisierten Prothesenwerkstoffes auf. Vorzugweise liegt die Brechzahl der Kern-Schale-Partikel um 1,4900 mit einer Schwankungsbreite von plus/minus 0,02, insbesondere +/- 0,01. Erfindungsgemäss besonders bevorzugte Kern-Schale-Partikel liegen aggregiert vor. Dabei weisen die Aggregate der Kern-Schale-Partikel, die regellos geformt sein können, als unregelmässig geformtes Aggregat einen mittleren Durchmesser d₅₀ ~ 50 - 300 µm auf. Die bevorzugte Grösse der Primärteilchengrösse beträgt kleiner 500 nm, insbesondere bis 100 nm, bevorzugt von 200 - 400 nm. Gleichfalls können Kern-Schale-Partikel mit einer Primärpartikelgrösse von kleiner gleich 200 nm bis 2 nm, wie zwischen 150 bis 10 nm als Kern-Schale-Partikel eingesetzt werden.

Vorzugsweise weisen die Kern-Schale-Partikel einen Brechungsindex von 1,48 bis 1,60 auf, insbesondere von 1,49 bis 1,55. Besonders bevorzugt liegt der Brechungsindex der Kern-Schale-Partikel im Bereich des Brechungsindexes von PMMA, PEMA, vorzugsweise liegt der Brechungsindex daher um 1, 48 bis 1,50.

Gleichfalls bevorzugt sind Kern-Schale-Partikel deren Dichte bei 0,9 bis 1,5 g/ml, insbesondere von 0,95 bis 1,4 g/ml, liegt. Vorzugsweise liegt die Schüttdichte zugleich bei 0,1 bis 0,6 g/ml.

Unter einer harten Aussenschale wird ein Material verstanden, das vorzugsweise eine geringere Elastizität als das Material der elastischen Phase aufweist. Vorzugsweise ist die Elastizität der harten Materialien mindestens 40% geringer als die der elastischen Phase. Bevorzugte anorganische harte Kerne zeigen unter dem Einfluss einer Kraft im Wesentlichen keine Verformung, während die organischen harten Materialien unter Einwirkung einer Kraft eine deutlich geringere Verformung erleiden als die elastische Phase. Die harten Materialien als harte Aussenschale stabilisieren die elastische Phase in ihrer Form. Eine elastische Phase wird aus mindestens einem elastischen Material gebildet, das unter der Einwirkung einer Kraft eine reversible Verformung erfährt. Die Verformung der elastischen Phase ist vorteilhaft ohne Krafteinwirkung vollständig reversibel.

Besonders bevorzugt umfasst die pulverförmige Komponente B) Polymethylmethacrylat (PMMA) Perlen als polymere Partikel und/oder Splitterpolymerisate, insbesondere mit Teilchengrössen von 10 - 100 µm, und/oder basiert auf Co-Polymeren umfassend einpolymerisierte Comonomere Styrol, alpha-Methylstyrol, Vinyltoluol, substituierte Vinyltoluole wie Vinylbenzylchloride, Vinylhalogenide wie Vinylchlorid, Vinylester, wie Vinylacetat, heterocyclische Vinylverbindungen wie 2-Vinylpyridin, Vinylacetat und Vinylpropionat, Butadien, Isobutylen, 2-Chlorbutadien, 2-Methylbutadien, Vinylpyridin, Cyclopenten, (Meth)acrylsäureester, wie Methylmethacrylat, Ethylmethacrylat,Butylmethacrylat, Butylacrylat und Hydroxyethylmethacrylat, ferner Acrylnitril, Maleinsäure und Maleinsäurederivate, wie beispielsweise Maleinsäureanhydrid, Fumarsäure und Fumarsäurederivate wie Fumarsäureester, Acrylsäure, Methacrylsäure sowie Aryl(meth)acrylate wie Benzylmethacrylat oder Phenylmethacrylat sowie optional Mischungen dieser Comonomere.

Optional kann die pulverförmige Komponente zusätzlich aufweisen: b) anorganische Füllstoffe umfassend pyrogene oder Fällungskieselsäuren, Dentalgläser wie Aluminosilicatgläser oder Fluoroaluminosilicatgläser, Bariumaluminiumsilicat, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis, insbesondere Mischoxide von SiO₂ und ZrO₂, Glasfasern und/oder Kohlenstofffasern sowie Mischungen umfassend die pulverförmigen Komponenten a) und b).

Die b) anorganischen Füllstoffe werden in der Regel zu 0 bis 10 Gew.-%, vorzugsweise von 0,0001 bis 3 Gew.-%, bezogen auf die gesamte Prothesenkunststoffzusammensetzung bzw. die Summe der Komponenten (A) und (B) eingesetzt. In der Komponente (B) in Bezug auf die Gesamtzusammensetzung der Komponente (B) von 100 Gew.-% liegen sie in der Regel im Bereich von 0 bis 20 Gew.-% vor, vorzugsweise 0,001 bis 10 Gew.-%.

Ebenfalls im Sinne der Erfindung sind polymere Partikel, die auf mindestens einem (Meth-)-acrylat Monomer mit nur einer (Meth-)acrylat-Gruppe basieren oder die auf der Mischung mindestens zweier dieser (Meth-)acrylat Monomere basieren.

Erfindungsgemässe Kern-Schale-Partikel umfassen als elastische Phase vorzugsweise mindestens ein Poly-(n-butyl-acrylat) PBA, Butadien-Styrol-Copolymer, Nitril-Butadien-Copolymer, Silikonkautschuk -(Pfropfcopolymerisat), Polyurethanpolymerisat, Polyolefinbasiertes Polyurethan (Polybutadien-basiertes Polyurethan), das vorzugsweise in MMA vorliegen kann. Die Partikelgrösse der Kern-Schale-Partikel kann kleiner gleich 500 nm, wie zwischen 50 nm bis 500 nm, insbesondere kleiner gleich 400 nm bis 100 nm, oder alternativ kleiner 100 nm bis 2 nm betragen, gleichfalls kann die elastische Phase auf Polydimethylsiloxan modifiziertem Polyurethanen und/oder Epoxy-funktionalisierten elastischen Phasen basieren.

Erfindungsgemässe Kern-Schale-Partikel umfassen als harte Schale mindestens ein (Meth)acrylat Polymer, vorzugsweise ein Alkyl(meth)acrylat-Polymer, wie PMMA; Polystyrol, einem Epoxy-funktionalisierten Kern, sowie Homo- oder Co-Kondensate der vorgenannten Polymere.

Besonders bevorzugte Kern-Schale-Partikel verleihen dem auspolymerisierten Prothesenmaterial im Fräsrohling in Kombination mit dem Urethan(meth)acrylat High Impact Eigenschaften, mit einer Bruchzähigkeit von ≥ 1,9 MPa · m^{1/2}, vorzugsweise ≥ 2 MPa · m^{1/2} und eine Gesamtbrucharbeit von ≥ 900 J/m². Bevorzugte Kern-Schale-Partikel umfassen Aggregate mit d₅₀ < 400 µm und Primärpartikelgrössen von d₅₀ kleiner 500 nm. Weiter bevorzugt können die Primärpartikel der Kern-Schale-Partikel grösser gleich 100 nm sein, insbesondere als d₅₀-Wert.

Gleichfalls geeignete Kern-Schale-Partikel umfassen einen elastischen Kern umfassend AcrylatPolymere mit harter Aussenschale, insbesondere mit einer Partikelgrösse kleiner 1 Micrometer.

Weiter bevorzugt weisen die Kern-Schale-Partikel gegenüber polymerisierbaren Monomeren reaktive Gruppen auf, vorzugsweise ist die Aussenschale mit (Meth)acrylat-Gruppen funktionalisiert. Alternative Kern-Schale-Partikel umfassen als festen Kern ein Siliciumdioxid und eine elastische Schale umfassend mindestens ein Nitril-Butadien-Copolymer. Weitere Kern-Schale-Partikel können in einer Monomerflüssigkeit vorliegen.

Als Urethan(meth)acrylate eignen sich erfindungsgemäss vorzugsweise difunktionelle und mehrfachfunktionelle Urethan(meth)acrylate, wie insbesondere Urethandi(meth)acrylate, besonders bevorzugt ist das mindestens eine (iii) Urethandimethacrylat (UDMA) ausgewählt aus linearen oder verzweigten Alkyl-funktionalisierten Urethandimethacrylaten, Urethandimethacrylat funktionalisierten Polyethern, insbesondere bis(methacryloxy-2-ethoxycarbonylamino)-alkylen, Bis(methacryloxy-2-ethoxycarbonylamino) substituierter Polyether, vorzugsweise 1,6-bis(methacryloxy-2-ethoxycarbonylamino)-2,4,4-trimethylhexane. Geeignete Urethan(meth)acrylate sind unter den folgenden Markennamen erhältlich: Ebecryl 230 (aliphatisches Urethandiacrylat), Actilane 9290, Craynor 9200 (Di-Urethanacrylat Ooligomer), Ebecryl 210 (aromatische Urethan-diacrylat Oligomere), Ebecryl 270 (aliphatische Urethandiacrylat Oligomer), Actilane 165, Actilane 250, Genomer 1122 (monofunktionelles Urethan- acrylat), Photomer 6210 (cas no. 52404-33-8, aliphatisches Urethan-diacrylat), Photomer 6623 (hexafunctional aliphatic Urethan Resin), Photomer 6891 (aliphatisches urethantriacrylat), UDMA, Roskydal LS 2258 (Aliphatisches Urethan-acrylat Oligomer), Roskydal XP 2513 (ungesättigtes aliphatisches Urethanacrylat).

Erfindungsgemäß bevorzugt ist ferner ein Fräsrohling umfassend Prothesenmaterial basierend auf der Umsetzung mindestens einer (A) flüssigen Monomerkomponente und einer (B) pulverförmigen Komponente, wobei die (A) flüssige Monomerkomponente, mindestens ein Monomer, insbesondere eine Mischung von Monomeren umfasst von a) Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornyl-acrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, Benzyl-, Furfuryl- oder Phenyl(meth)acrylat eine Mischung enthaltend mindestens eines dieser (Meth-)acrylate und/oder Co-Polymere umfassend eines oder mindestens zwei der vorgenannten Monomere, und/oder b) zwei- und/oder Mehrfachvernetzer (>2) umfassend 1,4-Butandiol-dimethacrylat (1,4-BDMA) oder Pentaerythritol-tetraacrylat, Bis-GMA-Monomer (Bisphenyl-A-Glycidyl-Methacrylat), Triethylen-glycoldimethacrylat (TEGDMA) und Diethylenglycoldimethacrylat (DEGMA), Tetraethylen-glykoldi(meth)acrylat, Decandioldi(meth)acrylat, Dodecandioldi(meth)acrylat, Hexyldecan-dioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate eine Mischung enthaltend mindestens eines dieser (Meth-)acrylate und/oder Co-Polymere umfassend eines oder mindestens zwei der vorgenannten Monomere.

Als geeignete Alkylmethacrylate für die flüssige Komponente (A) sei auf Methyl-, Ethyl-, n-Propyl, i-Propyl-, n-Butyl, t-Butyl, i-Butyl, Benzyl- und Furfurylmethacrylate oder deren Mischungen verwiesen. Methylmethacrylat ist davon besonders bevorzugt.

Erfindungsgemäß umfasst die (A) flüssige Monomerkomponente a.1) von > 85 Gew.-% mindestens eine monofunktionelle (Meth)acrylat-funktionelle Monomerkomponente, insbesondere 85 bis 90 Gew.-%, a.2) von 0 - 15 Gew.-% mindestens eine difunktionelle Di(meth)acrylat-funktionelle Monomerkomponente, insbesondere 0,01 bis 15 Gew.-%, 1 bis 10 Gew.-%, und a.3) von 0 - 10 Gew.-% mindestens ein (meth)acrylat-funktionelles trifunktionelles (Meth)acrylat oder ein mehrfachfunktionelles (Meth)acrylat auf, insbesondere 0,01 bis 10 Gew.-%, vorzugsweise 1,0 bis 8 Gew.-%, b) von 0 - 5 Gew.-% Stabilisatoren und Aktivatoren, Initiatoren, insbesondere 0,01 bis 5 Gew.-%, vorzugsweise 0,1 bis 3 Gew.-% c) von 0,001 bis 20 Gew.-% Urethan(meth)acrylat, insbesondere 0,01 bis 10 Gew.-%, bevorzugt von 0,5 bis 5 Gew.-%, besonders bevorzugt von 0,01 bis 3 Gew.-%, d) von 0,001 bis 10 Gew.-% Kern-Schale Partikel, bevorzugt 1 bis 10 Gew.-%, besonders bevorzugt 0,001 bis 5 Gew.-%, vorzugsweise zwischen 0,5 bis 5 Gew.-%. wobei die Gesamtzusammensetzung der flüssigen Monomerkomponente A) 100 Gew.-% beträgt. Gleichfalls beträgt die Gesamtzusammensetzung aller Komponenten der pulverförmigen Komponente (B) 100 Gew.-%. Die Kern-Schale-Partikel werden in der Regel in den Monomeren suspendiert.

Erfindungsgemäss setzt sich die Gesamtzusammensetzung der pulverförmigen Komponente (B) wie folgt zusammen: b.1) von 100 Gew.-% polymere Partikel und optional anorganische Füllstoffe oder ein Gemisch dieser, der Gehalt kann sich vorzugsweise wie folgt zusammensetzen von 100 bis 80 Gew.-% polymere Partikel, wie PMMA, PEMA, von 0 bis 20 Gew.-% anorganische Füllstoffe, wie Dentalgläser, Metalloxid- oder Mischoxidbasis (SiO₂, ZrO₂ und/oder TiO₂), insbesondere 0,01 bis 10 Gew.-%, b.2) von 0 - 5 Gew.-% zumindest einen Teil eines Initiatorsystems, insbesondere 0,01 bis 4 Gew.-%, und b.3) von 0 - 5 Gew.-% Pigmente, Hilfsstoffe, Stabilisatoren oder Gemische umfassend mindestens eine der vorgenannten Komponenten, insbesondere 0,01 bis 4 Gew.-%, vorgenannte Gesamtzusammensetzung der pulverförmigen Komponente (B) beträgt 100 Gew.-%.

Bevorzugt können pulverförmige polymere Komponenten, wie PMMA, unterschiedlicher Partikelgrössen ggf. als Homo- und/oder Co-Polymere eingesetzt werden: a) Homo-Polymer Perle 1 (dso ~ 40 - 50 µm) und b) Perle 2 (dso ~ 55 - 70 µm), und optional Co-Polymer c) Perle 3 (dso ~ 40 - 50 µm) eingesetzt werden.

Die Gesamtzusammensetzung (A) mit 100 Gew.-% und die Gesamtzusammensetzung (B) mit 100 Gew.-% werden dann im Gewichtsverhältnis von (A) zu (B) von 1 zu 20 bis 20 zu 1 gemischt, bevorzugt im Gewichtsverhältnis von (A) zu (B) von 5 zu 15 bis 9 zu 8, bevorzugt von 5 bis 8 zu 8 bis 12, vorzugsweise im Gewichtsverhältnis von (A) zu (B) von 7 zu 10, insbesondere mit einer Schwankungsbreite von plus/minus 1, vorzugsweise von 0,5.

Als weitere flüssige Monomere kommen die auf dem Dentalgebiet üblichen Monomere in Betracht: Beispiele sind radikalisch polymerisierbare monofunktionelle Monomere wie Mono(meth)-acrylate, Methyl-, Ethyl-, Butyl-, Benzyl-, Furfuryl- oder Phenyl(meth)acrylat.

Typische difunktionelle Monomere, auch als Vernetzer bzw. Mehrfachvernetzer bezeichnet, sind BDMA, 1,4-Butandiol-dimethacrylat (1,4-BDMA), Bis-GMA-Monomer (Bisphenyl-A-Glycidyl-Methacrylat, (ein Additionsprodukt aus Methacrylsäure und Bisphenol-A-diglycidyl-ether), Diethylenglykoldi(meth)acrylat, Bisphenol-A-di(meth)acrylat, Decandioldi(meth)acrylat, Dodecandioldi(meth)acrylat, Hexyldecandioldi(meth)acrylat, sowie Butandioldi(meth)acrylat, Ethylenglycol-di(meth)acrylat, Polyethylenglycol-di(meth)acrylate, ethoxylierte/propoxylierte Bisphenol-A-di(meth)acrylate. Die folgenden difunktionellen Monomere können auch als Verdünnungsmittel (dünnflüssige Acrylate wie Triethylenglycoldimethacrylat (TEGDMA) und Diethylenglycoldimethacrylat (DEGMA), zugesetzt werden. Tri- und tetraafunktionelle Monomere bzw. Mehrfachvernetzer umfassen Tri- oder Tetraethylenglykoldi(meth)acrylat, Trimethylolpropan-tri(meth)acrylat, tris(2-Hydroxyethyl)-isocyanurat-triacrylat, Pentaerythritol-tetraacrylat, Weitere Vernetzer sind nachfolgend auch unter den polymeren Partikeln umfassend Co-Polymere offenbart, die mindestens ein (Meth-)acrylat Monomer mit zwei, drei, vier, fünf oder sechs (Meth-)acrylat-Gruppen umfassen.

Der Anteil an bei Raumtemperatur flüssigem aliphatischem (Meth)acrylat im erfindungsgemässen, gemischten, noch nicht auspolymerisiertem Prothesenbasismaterial, insbesondere umfassend die Komponenten (A) und (B), beträgt beispielsweise 0,5 % bis 40 Gew.%, bevorzugt 20 bis 40 Gew.% Das aliphatische (Meth)acrylat kann in der flüssigen Monomerkomponente (A) oder optional zumindest teilweise in der Feststoffkomponente bzw. Pulverkomponente (B) oder in beiden zugegen sein. Bevorzugt befindet es sich in Komponente (A).

Ferner ist erfindungsgemäß bevorzugt ein Fräsrohling aus einem Prothesenmaterial, das vorzugsweise in Komponente (A), (B) oder in (A) und (B) zusätzlich mindestens einen oder mehrere Stoff(e) aus den Gruppen der Füllstoffe, Pigmente, Stabilisatoren, Regler, antimikrobiellen Additive, UV-Absorber, Thixotropiermittel, Katalysatoren und Vernetzer enthält. Solche Additive liegen - wie auch Pigmente, Stabilisatoren und Regler - in eher geringen Mengen im Fräsrohling vor, z. B. insgesamt zu 0,01 bis 3,0, besonders 0,01 bis 1,0 Gew.% bezogen auf die Gesamtmasse des Materials. Geeignete Stabilisatoren sind z. B. Hydrochinonmonomethylether oder 2,6-Di-tert.-butyl-4-methylphenol (BHT).

Offenbart wird gleichfalls ein polymerisierbares Prothesenmaterial zur Herstellung der auspolymerisierten Fräsrohlinge, das optional zusätzlich mindestens einen Initiator oder mindestens ein Initiatorsystem für die Autopolymerisation oder Heisspolymerisation aufweist, das je nach Reaktionsbedingungen bzw. Polymerisationssystem, in der flüssigen Komponente (A), der pulverförmigen Komponente (B) oder in (A) und (B) vorliegen.

Die folgenden Initiatoren und/oder Initiatorsysteme für die Auto- oder Kaltpolymerisation umfassen a) mindestens einen Initiator insbesondere mindestens ein Peroxid und/oder Azoverbindung, insbesondere LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid, und optional b) mindestens einen Aktivator, insbesondere mindestens ein aromatisches Amin, wie N,N-Dimethyl-p-toluidin, N,N-Dihydroxyethyl-p-toluidin und/oder p-Dibenzylaminobenzoesäurediethylester oder c) mindestens ein Initiatorsystem ausgewählt aus Redoxsystemen, insbesondere eine Kombinationen ausgewählt aus Dibenzoylperoxid, Dilauroylperoxid und Campherchinon mit Aminen ausgewählt aus N,N-Dimethyl-p-toluidin, N-N-Dihydroxyethyl-p-toluidin und p-Dimethylaminobenzoesäurediethylester oder ein Redoxsystem umfassend ein Peroxid, und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend (i) Barbitursäure oder Thiobarbitursäure oder ein Barbitursäure- oder Thiobarbitursäurederivat und (ii) mindestens ein Kupfersalz oder Kupferkomplex und (iii) mindestens eine Verbindung mit einem ionischen Halogenatom, besonders bevorzugt ist ein Redoxsystem umfassend 1-Benzyl-5-Phenylbarbitursäure, Kupferacetylacetonat und Benzyldibutylammoniumchlorid. Besonders bevorzugt wird die Polymerisation im 2-Komponenten Prothesenbasismaterial über ein Barbitursäurederivat gestartet.

Als Initiatoren für die Heisspolymerisation können die bekannten Peroxide wie Dibenzoylperoxid, Dilauroylperoxid, tert.-Butylperoctoat oder tert.-Butylperbenzoat eingesetzt werden, aber auch alpha, alpha '-Azo-bis(isobutyroethylester), Benzpinakol und 2,2'-Dimethylbenzpinakol sind geeignet.

Als Photoinitatoren kommen z.B. Benzoinalkylether oder -ester, Benzilmonoketale, Acylphosphinoxide oder aliphatische und aromatische 1.2-Diketoverbindungen, wie beispielsweise 2,2-Diethoxyacetophenon 9,10-Phenanthrenchinon, Diacetyl, Furil, Anisil, 4,4'-Dichlorbenzil und 4,4'-Dialkoxybenzil oder Campherchinon, in Frage. Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Heisspolymerisation eingesetzt werden.

Es können auch dual härtende Systeme verwendet werden, z. B. Photoinitiatoren mit Aminen und Peroxiden. Die Initiatoren werden vorzugsweise in Mengen von 0,01 bis 1 Gew.% bezogen auf die Gesamtzusammensetzung verwendet.

Offenbart wird auch ein Verfahren zur Herstellung eines Fräsrohlings umfassend auspolymerisiertes Prothesenmaterial, sowie ein Fräsrohling oder dreidimensionaler Formkörper erhältlich nach dem Verfahren, indem die Komponenten A) mindestens eine flüssige Monomerkomponente, und B) mindestens eine pulverförmige Komponente gemischt werden und nachfolgend auspolymerisiert bzw. gehärtet werden.

Besonders bevorzugt umfasst A) die flüssige Monomerkomponente Methylmethacrylat, Butandiolmethacrylat und optional mindestens ein methacrylat-basiertes di-, tri- und oder tetrafunktionelles Monomer als Mehrfachvernetzer, wie beispielsweise tris(2-Hydroxyethyl)-isocyanurat-triacrylat und/oder Pentaerythritol-tetraacrylat, sowie ein Urethandiacrylat, zumindest ein Teil eines Initiatorsystems und B) umfasst PMMA Perlen, Barbitursäure oder ein Barbitursäurederivat, sowie optional Pigmente. Zur Herstellung des Prothesenbasismaterials werden die Komponenten A) und B) gemischt.

Nach einer bevorzugten Verfahrensvariante werden die A) Monomerkomponente und die B) pulverförmige Komponente im Gewichtsverhältnis von 1 : 50 bis 50 : 1, insbesondere im Gewichtsverhältnis 7 : 10 (150 g Pulver zu 105 ml Flüssigkeit oder 10 g Pulver, 7 ml Flüssigkeit, mit ähnlicher Dichte) gemischt, insbesondere mit einer Schwankungsbreite von plus/minus 1, bevorzugt plus/Minus 0,5. Das Verhältnis Pulver zu Flüssigkeit liegt bei etwa 1,35 bis 1,5 zu 1, vorzugsweise beträgt das Gewichtsverhältnis etwa 1,4 - 1,5 zu 1 von Pulver zu Flüssigkeit.

Das Mischen der Komponenten A) und B) kann erfindungsgemäss mittels einfacher dem Zahntechniker bekannter Massnahmen erfolgen, wie mittels Spatel.

Ebenso ist erfindungsgemäß bevorzugt ein pigmentierter Fräsrohling aus auspolymerisiertem Prothesenmaterial. Ferner ist erfindungsgemäß bevorzugt ein Fräsrohling aus unpigmentiertem, auspolymerisiertem Prothesenmaterial mit einer Transparenz von grösser 85 %, insbesondere größer 90 % (gemessen an 3 mm Platten mit einer Höhe von 3mm).

Nach einer weiteren Ausgestaltung wird offenbart die Verwendung von mit mindestens einer elastischen Phase modifizierten Kern-Schale-Partikeln und mindestens einem Urethandi(meth)acrylat oder einem Derivat eines Urethandi(meth)acrylats in auto- oder kaltpolymerisierbaren Prothesenmaterialien zur Herstellung von Fräsrohlingen.

Ebenso wird offenbart die Verwendung von mit mindestens einer elastischen Phase modifizierten Kern-Schale-Partikeln in Prothesenmaterialien und optional mindestens ein Urethandimethacrylat enthaltenden Prothesenmaterialien zur Herstellung von Fräsrohlingen oder Halbfertigprodukten oder Halbfertigproduktformen von Fräsrohlingen, insbesondere zur Herstellung von dentalen Fräsrohlingen oder orthopädischen Fräsrohlingen. Dabei ist die Herstellung von CNC- und/oder CAD/CAM Fräsrohlingen besonders bevorzugt.

Ferner ist Gegenstand der Erfindung die Verwendung eines erfindungsgemäßen Fräsrohlings, insbesondere mit mindestens einer elastischen Phase modifizierten Kern-Schale-Partikeln und optional umfassend ein Urethandimethacrylat, zur Herstellung von Komponenten, insbesondere von dentalen oder orthopädischen Komponenten, umfassend Prothesenbasisplatten, Aufbissschienen, Kronen, Brücken, künstlichen Zähnen, Verblendschalen, Inlays, Onlays, kiefer-orthopädischen Apparaten, aktiven oder passiven kiefer-orthopädischen Apparaturen, Crozat-Apparaturen, modifizierten Aktivatoren, Implantaten, Superstrukturen, dentalen Stegen, Abutments, dentalen Befestigungsmitteln, dentalen Schrauben, Bohrschablonen für die Implantologie, Teleskope, Veneers, Mouthgards, künstlichen Gelenkprothesen, Zahnspangen, Vorschubdoppelplatten, Implantatteilen, unsichtbaren Zahnspangen, Brackets, Multibracket-Apparaturen, kieferorthopädische Instrumente und/oder Multiband-Apparaturen oder zumindest von Teilen der vorgenannten Komponenten, oder im Veterinärbereich, insbesondere für Hufreparaturkomponenten.

Nach einer weiteren Alternative wird offenbart ein Kit umfassend ein autopolymerisierbares Prothesenmaterial, wobei das Kit separierte Komponenten (A) und (B) umfasst zur Herstellung von Fräsrohlingen.

Als auto- oder kaltpolymerisierbar gilt ein Prothesenmaterial gemäss der Erfindung, wenn die Kriterien nach ISO 20795-1 (Pkt. 3.1) erfüllt sind. Als kaltpolymerisierende Kunststoffe gelten Zusammensetzungen, die unterhalb 65 °C polymerisieren. Erfindungsgemässe kaltpolymerisierende Prothesenmaterialien können vorzugsweise in einem Temperaturbereich nach Mischen der beiden Komponenten (A) und (B) von 50 °C bis 65 °C, vorzugsweise von 50 bis 60 °C, weiter bevorzugt von 50 °C bis 55 °C selbständig aushärten bzw. auspolymerisieren. Entsprechend der vorstehenden Norm werden polymerisierbare Zusammensetzungen, die ab 65 °C selbständig aushärten bzw. auspolymerisieren, als heisshärtende Zusammensetzungen, bezeichnet.

Die Pulverkomponente des Zweikomponenten-Prothesenbasismaterials enthält in der Regel ein Polymerpulver, insbesondere auf Methacrylatbasis, und/oder ein Perlpolymerisat auf Methacrylat-Basis. Perlpolymerisate werden auf diesem Gebiet häufig als Pulver bezeichnet.

Perlpolymerisate, insbesondere solche aus (Meth)acrylaten, sind dem Fachmann bekannt. Perlpolymerisate auf der Basis von Polyalkyl(meth)acrylaten erhält man in bekannter Weise durch Fällungspolymerisation oder Suspensionspolymerisation. Dabei liefert die Suspensionspolymerisation in der Regel grössere Teilchen. Die durchschnittlichen Teilchengrössen decken eine weite Spanne ab und können beispielsweise von 0,1 µ bis 250 µm betragen. Es kann sich auch um vernetzte Perlpolymerisate handeln. Geeignete multifunktionelle Vernetzermoleküle sind der obigen Aufzählung der auf dem Dentalgebiet üblichen Monomeren zu entnehmen. In das Perlpolymerisat können auch weitere Comonomere, wie vorstehend erläutert, einpolymerisiert sein.

In einer bevorzugten Ausgestaltung sind in die Perlen des ersten (Co)polymerisats und/oder in die Perlen des zweiten (Co)polymerisats zumindest teilweise Vernetzer einpolymerisiert worden. Die ersten und zweiten Perlpolymerisate umfassen somit auch vernetzte und teilvernetzte Perlpolymerisate.

Für die Vernetzung greift man regelmässig auf multifunktionelle Comonomere oder auch multifunktionelle Oligomere zurück. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, ausserdem Glycerindi(meth)acrylat, 2,2-bis[(gamma-methacryloxy-beta-oxypropoxy)-phenylpropan], bis-GMA, Bisphenol-A-dimethacrylat, Neopentylglycol-di(meth)acrylat, 2,2-di-methacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie di-oder trivinylische Verbindungen wie Divinylbenzol hervorgehoben.

Der Gehalt an derartigen Vernetzermolekülen liegt vorzugsweise im Bereich von 0,1 Gew.-% bis 10 Gew.-%, insbesondere im Bereich von 0,5 Gew.-% bis 5 Gew.-%, in der Ausgangsmischung für das Perlpolymerisat.

In einer zweckmässigen Ausgestaltung liegt in der flüssigen Komponente (A) neben mindestens einem der vorangehend genannten monofunktionellen Alkylmethacrylatmonomere mindestens ein Vernetzer vor. Dabei handelt es sich beispielsweise um multifunktionelle Monomere, Comonomere oder auch multifunktionelle Oligomere. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, ausserdem Glycerindi(meth)acrylat, 2,2-bis[(gamma-methacryloxy-beta-oxypropoxy)-phenylpropan], bis-GMA, Bisphenol-A-dimethacrylat, Neopentylglycol-di(meth)acrylat, 2,2-di-methacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie di-oder trivinylische Verbindungen wie Divinylbenzol hervorgehoben. Selbstverständlich können auch Mischungen der genannten Vernetzermoleküle zum Einsatz kommen. Besonders geeignet sind auch solche multifunktionellen Verbindungen, insbesondere di- und/oder tri-funktionelle Verbindungen, die elastische Einheiten besitzen und demgemäss geeignet sind, die aus den Prothesenausgangsmaterialien erhaltenen Prothesenmaterialien mit flexiblen Eigenschaften auszustatten.

Exemplarisch sei auf die Dimethacrylate, wie 1,4-Butandiol-dimethacrylat, verwiesen. Solche Vernetzermoleküle können in der flüssigen Monomerkomponente (A) in Mengen im Bereich von 0,1 bis 20 Gew.-%, vorzugsweise im Bereich von 1 bis 10 Gew.-%, beispielsweise 5 Gew.-% zugegen sein.

In einer weiteren Ausführungsform können in der Flüssigkomponente (A) neben z. B. Methylmethacrylat als bevorzugtem Hauptmonomer (> 50 Gew.-%) weitere Comonomere vorliegen.

Das für die Polymerisation erforderliche radikalische Initiatorsystem ist, je nach Reaktionsbedingungen bzw. Polymerisationssystem, in der flüssigen Komponente (A) und/oder der pulverförmigen Komponente (B) enthalten. Diesbezügliche Details sind dem Fachmann bekannt. Beispielsweise liegt bei Basismischungen für Kaltpolymerisate das Initiatorsystem meist in beiden Komponenten, der flüssigen Komponente und der pulverförmigen Komponente vor und wird demgemäss beim Mischen dieser Komponenten zusammengeführt. Folglich liegt in der Regel eine Initiatorkomponente (c) in der pulverförmigen Komponente (B) vor, insbesondere in Form von Peroxiden, Perketalen, Perestern und/oder Azoverbindungen. Ein weiterer Teil des Initiatorsystems (c) kann sich in der Flüssigkomponente (A) befinden, in der Regel ein Coinitiator. Als Initiatoren können auch Restgehalte an bei der Herstellung der pulverförmigen Komponenten nicht abreagierter Initiatorkomponente verwandt werden, z. B. Peroxide wie Dibenzoylperoxid.

Als Initiatoren für die Polymerisationsreaktion von kalt- bzw. autopolymerisierenden Ausgangsmischungen kommen grundsätzlich solche in Betracht, mit denen sich radikalische Polymerisationsreaktionen starten lassen. Bevorzugte Initiatoren sind Peroxide sowie Azoverbindungen, wie zum Beispiel die folgenden: LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid.

Um die Initiierung der radikalischen Polymerisation durch Peroxide zu beschleunigen, können geeignete Aktivatoren, z. B. aromatische Amine, hinzugefügt werden. Exemplarisch seien als geeignete Amine N,N-Dimethyl-p-toluidin, N,N-Dihydroxyethyl-p-toluidin und p-Dibenzylaminobenzoesäurediethylester genannt. Hierbei fungieren die Amine regelmässig als Co-Initiatoren und liegen üblicherweise in einer Menge von bis zu 0,5 Gew.-% vor.

Als radikalische Initiatorsysteme sind die genannten Redoxsysteme geeignet. In einer zweckmässigen Ausführungsform enthält ein solches Redoxsystem Barbitursäure oder Thiobarbitursäure oder ein Barbitursäure- oder Thiobarbitursäurederivat (beispielsweise 25 bis 80 Gew.-%), mindestens ein Kupfersalz oder ein Kupferkomplex (beispielsweise 0,1 bis 8 Gew.-%) und mindestens eine Verbindung mit einem ionogen vorliegenden Halogenatom (beispielsweise 0,05 bis 7 Gew.-%). Exemplarisch seien als geeignete Bestandteile des vorangehend genannten Redoxsystems 1-Benzyl-5-Phenylbarbitursäure, Kupferacetylacetonat und Benzyldibutylammoniumchlorid genannt.

Die Aushärtung der polymerisierbaren Prothesenmaterialien erfolgt vorzugsweise durch redoxinduzierte radikalische Polymerisation bei Raumtemperatur bzw. bei leicht erhöhter Temperatur unter leichtem Druck, um Blasenbildung zu vermeiden. Als Initiatoren für die bei Raumtemperatur durchgeführte Polymerisation werden z.B. Redoxinitiator-Kombinationen, wie z.B. Kombinationen von Benzoyl- oder Laurylperoxid mit N,N-Dimethyl-sym.-xylidin oder N,N-Dimethyl-p-toluidin, verwendet. Ein besonders bevorzugtes Initiatorsystem ist eine Kombination von Barbitursäuren in Verbindung mit Kupfer- und Chlorid-ionen sowie oben genannten Peroxiden. Dieses System zeichnet sich durch eine hohe Farbstabilität aus.

Ferner kann man die pulverförmige Komponente (B) und/oder die Flüssigkomponente (A) in bekannter Weise mit weiteren Zusatzstoffen aus den Gruppen der Stabilisatoren, UV-Absorbern, Thixotropiermitteln und Füllstoffen versehen.

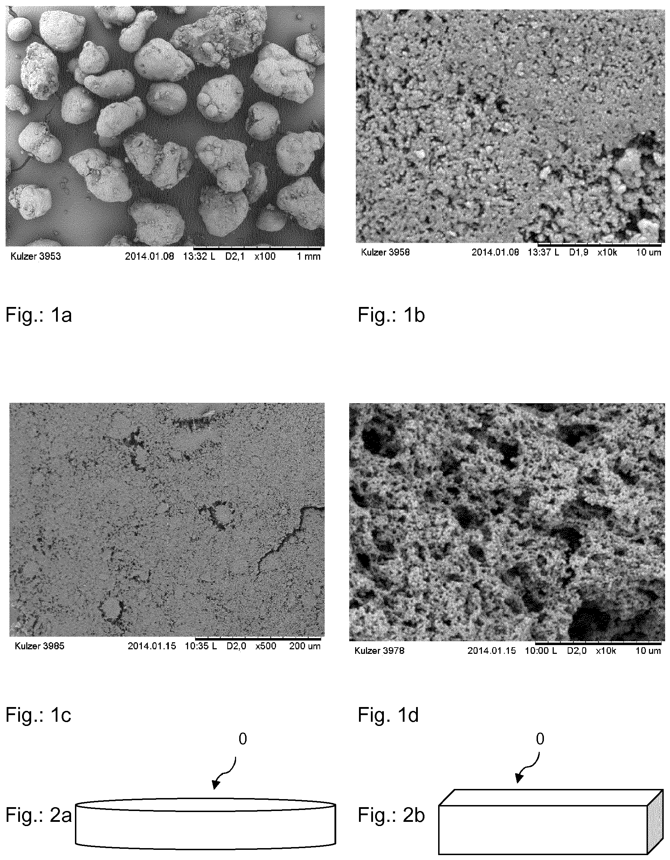

Die Figuren 1a bis d beschreiben den Erfindungsgegenstand näher, ohne ihn auf diese Ausführungsformen zu beschränken.

Wie vorstehend dargestellt kann der erfindungsgemäße Fräsrohling vorzugsweise zahnfarben, farblich der Gingiva nachgebildet sein oder bereichsweise mehrfarbig ausgebildet sein. Zur Einstellung der Zahnfarbe und/oder Farbe bzw. Farbverläufen der Gingiva können Farbpigmente dem polymerisierbaren Prothesenmaterial zugesetzt oder in dem Prothesenmaterial des Fräsrohlings enthalten sein:
Figur 1a und Figur 1b: REM - Aufnahmen der Kern-Schale-Partikel (links: 100x, rechts 10.000x); Figur 1c und d. REM - Aufnahme des in MMA suspendierten und vor der Aufnahme getrockneten Additivs (links: 500x, rechts 10.000x); Figuren 2a und 2b - Beispiele für Fräsrohlinge (0)

### Ausführungsbeispiel:

Herstellen der erfindungsgemässen Pulvermischung (B): Aus PMMA-Perlen unterschiedlicher Korngrösse (Perle 1 Homo-Polymerisat (d50 ~ 40 - 50 µm) 60 - 70 %, Perle 2 (d50 ~ 55 - 70 µm) 15 %, Perle 3 Co-Polymerisat (d50 ~ 40 - 50 µm) 15 %) wird unter Zusatz von Barbitursäure und Farbpigmenten eine Mischung hergestellt.

Herstellen der erfindungsgemässen Flüssigkeit/Monomermischung (A):
Aus Methylmethacrylat, und einem weiteren methacrylat-basierten Mehrfach-Vernetzer wird unter Zusatz von Stabilisatoren und Initiatoren (Barbitursäure / Kupfer - System) eine Mischung hergestellt. Ferner wird das erfindungsgemässe Urethandimethacrylat sowie der erfindungsgemässe Kern-Schale-Partikel zugesetzt.

Erfindungsgemässes Beispiel Pulver- und Flüssigkeitsmischung:

| **Flüssigkeit** | **Beispiel 1** |
|---|---|
| Methylmethacrylat | 93,3 |
| 2-(4,6-diphenyl-1,3,5-triazin-2-yl)-5-((hexyl)oxy)-phenol | 0,3 |
| N-Methyl-N,N-dioctyloctan-1-ammoniumchlorid | 0,2 |
| Kupfer(II) Chlorid Lsg. | 0,1 |
| N,N-Bis(2-hydroxyethyl)-p-toluidin | 0,1 |
| ges. | 94,00 |
| | |
| Difunktionales aliphatisches urethanacrylat oligomer | 3,00 |
| tris(2-hydroxy ethyl) isocyanurat-triacrylate | 1,00 |
| Kern-Schale Partikel | 2,00 |
| ges. | 100,00 |

| **Pulver** | |
|---|---|
| PMMA/PMA d₅₀ ~ 40-45 µm | 67,5 |
| PMMA d₅₀ ~ 40-55 µm | 15,000 |
| Vernetztes PMMA d₅₀ ~ 55-70 µm | 15,000 |
| Phenylbenzylbarbitursäure | 2,5 |
| ges. | 100,000 |

**Tabelle 1: Gegenüberstellung des erfindungsgemässen Beispiels und der Vergleichsbeispiele**

| Physikalische Eigenschaften (Norm ISO 20795-1) | | | |
|---|---|---|---|
| | | Bsp. 1 | PMMA |
| Biegefestigkeit [MPa] | >60 | 71,1 | 85,9 |
| E-Modul [MPa] | >1500 | 2389 | 2518 |
| Bruchzähigkeit [MPa m1/2] | >1,9 | 2,36 | 1,42 |
| Gesamtbrucharbeit [J/m²] | >900 | 1030,85 | 197,64 |
| Transparenz [delta %] (nach 6 d/ 37 °C in Ringerlösung) | | -3,16 | |

### Herstellung von Prüfkörpern, Bestimmung der Farbwerte, Bestimmung der mechanischen Eigenschaften:

**Farbprüfkörper:** Folgende Pulver- und Monomermischungen werden im Verhältnis

10 g Pulver : 7 ml Flüssigkeit intensiv gemischt und nach der Anquellphase (ca. 5 Min bei 23 °C) Prüfkörper mit einer Abmessung von 30 x 30 x 3 mm gegossen und 30 Min. bei 55°C und 2 bar Druck im Palamat elite auspolymerisiert.

**Prüfkörper für mechanische Festigkeit:** Folgende Pulver- und Monomermischungen werden im Verhältnis 150 g Pulver: 105 ml Flüssigkeit intensiv für etwa 30 Sekunden gemischt und nach der Anquellphase (ca. 3 Min. bei 23 °C) in eine Metallform gegossen. Die Metallform ist ein einseitig geschlossenes Rohr aus Messing mit einem Innendurchmesser von 102 mm und einer Höhe von 50 mm. Die Polymerisation erfolgt in einem Drucktopf Palamat elite über 30 Min. bei 55°C und 3,8 bar Druck.

Die Prüflinge werden aus dem erhaltenen Fräsrohling mittels CAD-/CAM entsprechend den Vorgaben der ISO 20795-1 heraus gefräst und abgeprüft.

Prüfkörper für farbmetrische Abprüfungen werden in Dubliergel hergestellt.

Zur Bestimmung der Transparenz werden die Prüfkörper für 5 Tage bei 37°C in Ringer-Lösung eingelagert und die Farbwerte und Transparenz an 3 mm-Platten jeweils vor und nach der Einlagerung bestimmt. Die Messungen der Tabelle 1 wurden gemäss der DIN EN ISO 20795-1 durchgeführt.

Die erfindungsgemässe Monomermischung zeigt eine deutlich verbesserte Bruchzähigkeit gegenüber allen Vergleichsbeispielen, eine erhöhte Transparenz sowie den geringsten Transparenzverlust nach einer Lagerung in einer Ringerlösung (reduzierte Neigung zu Weissverfärbung).

## Patentansprüche

1. Fräsrohling aus polymerem Prothesenmaterial basierend auf der Umsetzung mindestens einer (A) flüssigen Monomerkomponente und einer (B) pulverförmigen Komponente, wobei die (A) flüssige Monomerkomponente
größer 85 Gew.-% mindestens eine monofunktionelle (Meth)acrylat-funktionelle Monomerkomponente,
0,001 bis 20 Gew.-% Urethan(meth)acrylat, und
0,001 bis 10 Gew.-% durch eine elastische Phase modifizierte Kern-Schale-Partikel mit elastischer Phase als Kern in harter Aussenschale, wobei die Kern-Schale-Partikel Primärpartikel mit einer mittleren Partikelgröße von kleiner gleich d₅₀ ≤ 500 nm bis 10 nm aufweisen,
umfasst, wobei die Gesamtzusammensetzung der flüssigen Monomerkomponente (A) 100 Gew.-% beträgt,
**dadurch gekennzeichnet, dass** das Prothesenmaterial in Form eines dreidimensionalen Formkörpers geeignet als CAD/CAM-Fräsrohling vorliegt und mindestens ein Polymer umfassend mindestens ein einpolymerisiertes Urethan(meth)acrylat enthält und 0,001 bis 10 Gew.-% der durch eine elastische Phase modifizierten Kern-Schale-Partikel in Bezug auf die Gesamtzusammensetzung umfasst, wobei der Formkörper eine
Bruchzähigkeit von ≥ 1,9 MPa · m^{1/2} und eine Gesamtbrucharbeit von ≥ 900 J/m² aufweist.

2. Fräsrohling nach Anspruch 1, **dadurch gekennzeichnet, dass** die Kern-Schale-Partikel Aggregate von Primärpartikel umfassen, wobei die Aggregate eine mittlere Partikelgrösse von kleiner gleich (d₅₀) 400 µm (Micrometer) aufweisen.

3. Fräsrohling nach einem der Ansprüche 1 oder 2, umfassend iii) 0,001 bis 20 Gew.-% mindestens ein einpolymerisiertes Urethan(meth)acrylat, insbesondere ein Urethandimethacrylat, in Bezug auf die Gesamtzusammensetzung.

4. Fräsrohling nach einem der Ansprüche 1 bis 3, **dadurch gekennzeichnet, dass** das unpigmentierte Prothesenmaterial eine Transparenz von grösser gleich 90 % aufweist (gemessen an Platten mit einer Höhe von 3 mm).

5. Fräsrohling nach einem der Ansprüche 1 bis 4, **dadurch gekennzeichnet, dass** er zu grösser gleich 55 Gew.-% PMMA in Bezug auf die Gesamtzusammensetzung umfasst.

6. Verfahren zur Herstellung eines auspolymerisierten Prothesenmaterials in Form eines dreidimensionalen Formkörpers, insbesondere in Form eines Fräskörper, indem ein polymerisierbares Prothesenmaterial umfassend die Komponente (A) und/oder (B),
- wobei die Komponente
A) mindestens eine flüssige Monomerkomponente, und
B) mindestens eine pulverförmige Komponente ist, und
das polymerisierbare Prothesenmaterial in Komponente (A)
größer 85 Gew.-% mindestens eine monofunktionelle (Meth)acrylat-funktionelle Monomerkomponente,
0,001 bis 20 Gew.-% Urethan(meth)acrylat, und 0,001 bis 10 Gew.-% durch eine elastische Phase modifizierte Kern-Schale-Partikel mit elastischer Phase als Kern in harter Aussenschale, wobei die Kern-Schale-Partikel Primärpartikel mit einer mittleren Partikelgröße von kleiner gleich d₅₀ ≤ 500 nm bis 10 nm aufweisen umfasst, wobei die Gesamtzusammensetzung der flüssigen Monomerkomponente (A) 100 Gew.-% beträgt,
und die Komponenten (A) und (B)
-
(a) gemischt werden,
(b) in eine dreidimensionale Form überführt werden,
(c) und nachfolgend das polymerisierbare Prothesenmaterial auspolymerisiert wird.

7. Verfahren nach Anspruch 6, **dadurch gekennzeichnet, dass** die A) Monomerkomponente und die B) pulverförmige Komponente im Gewichtsverhältnis von 1 : 50 bis 50 : 1, insbesondere im Gewichtsverhältnis 8 bis 11 pulverförmige Komponente zu 5 bis 8 Monomerkomponente gemischt werden.

8. Verfahren nach Anspruch 6 oder 7, **dadurch gekennzeichnet, dass**
A) die flüssige Monomerkomponente mindestens ein Monomer oder eine Mischung von Monomeren umfasst von a) Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornyl-acrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, Benzyl-, Furfuryl- oder Phenyl(meth)acrylat eine Mischung enthaltend mindestens eines dieser (Meth-)acrylate und/oder Co-Polymere umfassend eines oder mindestens zwei der vorgenannten Monomere, und/oder
b) zwei- und/oder Mehrfachvernetzer umfassend 1,4-Butandiol-dimethacrylat (1,4-BDMA) oder Pentaerythritol-tetraacrylat, Bis-GMA-Monomer (Bisphenyl-A-Glycidyl-Methacrylat), Triethylenglycoldimethacrylat (TEGDMA) und Diethylenglycoldimethacrylat (DEGMA), Tetraethylenglykoldi(meth)acrylat, Decandioldi(meth)acrylat, Dodecandioldi(meth)acrylat, Hexyldecandioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate eine Mischung enthaltend mindestens eines dieser (Meth-)acrylate und/oder Co-Polymere umfassend eines oder mindestens zwei der vorgenannten Monomere, und/oder
B) die pulverförmige Komponente umfasst polymere Partikel umfassend Polymere in Form von Polymerpulver umfassend Polyalkyl(meth)acrylate, die optional vernetzt sind und als Homo- oder Co-Polymere vorliegen, wobei die Polymere auf mindestens einem der Monomere, umfassend eine (Meth-)acrylat-Gruppe, basieren, ausgewählt aus Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Methylacrylat, Ethylacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Hydroxyethylacrylat, Hydroxypropylacrylat, Hydroxyethylmethacrylat, Hydroxypropylmethacrylat, eine Mischung enthaltend mindestens eines dieser (Meth-)acrylate und/oder Co-Polymere umfassend eines oder mindestens zwei der vorgenannten Monomere, Polyamidpartikel, Polyamidfasern, besonders bevorzugt ist Polymethylmethacrylat (PMMA).

9. Auspolymerisiertes Prothesenmaterial erhältlich nach einem Verfahren nach einem der Ansprüche 6 bis 8, **dadurch gekennzeichnet, dass** das Prothesenmaterial in Form eines dreidimensionalen Fräskörpers vorliegt, insbesondere in Form eines Fräsrohlings.

10. Auspolymerisiertes Prothesenmaterial nach Anspruch 9, **dadurch gekennzeichnet, dass** es eine Bruchzähigkeit von ≥ 1,9 MPa · m^{1/2} und eine Gesamtbrucharbeit von ≥ 900 J/m² aufweist.

11. Verwendung eines Fräsrohlings nach einem der Ansprüche 1 bis 5, zur Herstellung von Komponenten, insbesondere dentalen oder orthopädischen Komponenten, umfassend Prothesenbasisplatten, Aufbissschienen, Kronen, Brücken, künstlichen Zähnen, Verblendschalen, Inlays, Onlays, kiefer-orthopädischen Apparaten, aktiven oder passiven kiefer-orthopädischen Apparaturen, Crozat-Apparaturen, modifizierte Aktivatoren, Implantaten, Superstrukturen, dentalen Stegen, Abutments, dentalen Befestigungsmitteln, dentalen Schrauben, Bohrschablonen für die Implantologie, Teleskope, Veneers, Mouthgards, künstlichen Gelenkprothesen, Zahnspangen, Vorschubdoppelplatten, Implantatteilen, unsichtbaren Zahnspangen, Brackets, Multibracket-Apparaturen, kiefer-orthopädische Instrumente und/oder Multiband-Apparaturen oder zumindest von Teilen der vorgenannten Komponenten, oder im Veterinärbereich, insbesondere für Hufreparaturkomponenten.

## Claims

1. Milling blank made of polymeric prosthetic material based on the reaction of at least
one (A) liquid monomer component and one (B) powdered component,
the (A) liquid monomer component comprising
greater 85 % by weight at least one mono-functional (meth)acrylate-functional monomer component,
0.001 to 20 % by weight urethane (meth)acrylate, and
0.001 to 10 % by weight core-shell particles modified by an elastic phase with elastic phase as core in solid outer shell, the core-shell particles having primary particles having an average particle size of less than or equal to d₅₀ ≤ 500 nm to 10 nm,
the total composition of the liquid monomer component (A) amounting to 100 % by weight,
**characterised in that** the prosthetic material is present in the form of a three-dimensional moulded body being suitable as CAD/CAM milling blank, and comprises at least one polymer comprising at least one urethane (meth)acrylate polymerised into the polymer, and 0.001 to 10 % by weight of the core-shell particles modified by an elastic phase, based on the total composition, the moulded body having a fracture toughness of ≥ 1.9 Mpa · m^{1/2} and a total fracture work of ≥ 900 J/m².

2. Milling blank according to claim 1, **characterised in that** the core-shell particles comprise aggregates of primary particles, the aggregates having an average particle size of less than or equal to (d₅₀) 400 µm (micrometers).

3. Milling blank according to any one of claims 1 or 2, comprising iii) 0.001 to 20 % by weight at least one urethane (meth)acrylate polymerised into the polymer, in particular a urethane dimethacrylate, based on the total composition.

4. Milling blank according to any one of claims 1 to 3, **characterised in that** the unpigmented prosthetic material has a transparency of greater than or equal to 90 % (measured against plates having a height of 3 mm).

5. Milling blank according to any one of claims 1 to 4, **characterised in** comprising greater than or equal to 55 % by weight PMMA, based on the total composition.

6. Method for producing a polymerised prosthetic material in the form of a three-dimensional moulded body, in particular in the form of a milling blank, in which a polymerisable prosthetic material comprising components (A) and/or (B)
- component
A) being at least one liquid monomer component, and
B) being at least one powdered component, and
the polymerisable prosthetic material comprising in component (A)
greater 85 % by weight at least one mono-functional (meth)acrylate-functional monomer component,
0.001 to 20 % by weight urethane (meth)acrylate, and
0.001 to 10 % by weight core-shell particles modified by an elastic phase with elastic phase as core in solid outer shell, the core-shell particles having primary particles having an average particle size of less than or equal to d₅₀ ≤ 500 nm to 10 nm, the total composition of the liquid monomer component (A) amounting to 100 % by weight,
and components (A) and (B)
-
(a) are being mixed,
(b) are being transferred into a three-dimensional mould,
(c) and the polymerisable prosthetic material is subsequently being polymerised.

7. Method according to claim 6, **characterised in that** the
A) monomer component and the B) powdered component are being mixed at a weight ratio of 1 : 50 to 50 : 1, in particular at a weight ratio of 8 to 11 powdered component to 5 to 8 monomer component.

8. Method according to claims 6 or 7, **characterised in that**
A) the liquid monomer component comprises at least one monomer or a mixture of monomers from a) methyl methacrylate, ethyl methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, polypropylene glycol monomethacrylate, methyl acrylate, ethyl acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofurylacrylate, polypropylene glycol monoacrylate, hydroxyethyl acrylate, hydroxypropyl acrylate, hydroxyethyl methacrylate, hydroxypropyl methacrylate, benzyl, furfuryl or phenyl (meth)acrylate, a mixture containing at least one of said (meth)acrylates and/or copolymers comprising one or at least two of the afore-mentioned monomers, and/or
b) two- and/or multi-crosslinking agents comprising 1,4-butanediol dimethacrylate (1,4-BDMA) or pentaerythritol tetraacrylate, bis-GMA monomer (bisphenol-A-glycidyl methacrylate), triethylene glycol dimethacrylate (TEGDMA) and diethylene glycol dimethacrylate (DEGMA), tetraethylene glycol di(meth)acrylate, decanediol di(meth)acrylate, dodecanediol di(meth)acrylate, hexyl decanediol di(meth)acrylate, trimethylolpropane tri(meth)acrylate, pentaerythritol tetra(meth)acrylate, as well as butanediol di(meth)acrylate, ethylene glycol di(meth)acrylate, polyethylene glycol di(meth)acrylates, ethoxylated/propoxylated bisphenol-A-di(meth)acrylate, a mixture containing at least one of said (meth)acrylates and/or copolymers comprising one or at least two of the afore-mentioned monomers, and/or
B) the powdered component comprises polymeric particles comprising polymers in the form of polymer powder comprising polyalkyl (meth)acrylates, optionally being crosslinked and being present as homopolymer or copolymers, the polymers being based on at least one monomer comprising a (meth)acrylate group, selected from methyl methacrylate, ethyl methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethyl acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate, a mixture containing at least one of said (meth)acrylates and/or copolymers comprising one or at least two of the afore-mentioned monomers, polyamide particles, polyamide fibers, particularly preferred is polymethyl methacrylate (PMMA).

9. Polymerised prosthetic material obtainable according to a method according to any one of claims 6 to 8, **characterised in that** the prosthetic material is present in the form of a three-dimensional milling body, in particular in the form of a milling blank.

10. Polymerised prosthetic material according to claim 9, **characterised in** having a fracture toughness of ≥ 1.9 MPa · m^{1/2} and a total fracture work of ≥ 900 J/m².

11. Use of a milling blank according to any one of claims 1 to 5, for producing components, in particular dental or orthopaedic components, comprising prosthesis base plates, occlusal splints, crowns, bridges, artificial teeth, coverings, inlays, onlays, orthodontic appliances, active or passive orthodontic appliances, Crozat appliances, modified activators, implants, superstructures, dental bars, abutments, dental fastening means, dental screws, surgical guides for implantology, telescopic prostheses and telescopic crowns, veneers, mouthguards, artificial joint protheses, braces, double bite jumping plates, implant part, invisible braces, brackets, multibracket appliances, orthodontic instruments and/or multiband appliances or of at least parts of the afore-mentioned components, or in the veterinary field, in particular for hoof repair components.

## Revendications

1. Ébauche de fraisage faite du matériau prothétique polymérique basé sur la réaction d'au moins un (A) composant monomère liquide et un (B) composant pulvérulent,
le (A) composant monomère liquide comprenant
supérieur à 85 % en poids au moins un composant monomère mono-fonctionnel à fonction méthacrylate,
0,001 à 20 % en poids (méth)acrylate d'urethane, et
0,001 à 10 % en poids des particules coeur-coque modifiées par une phase élastique avec la phase élastique au tant qu'un cœur dans une coque extérieure solide, les particules coeur-coque ayant des particules primaires ayant une dimension particulaire moyenne d'inférieure ou égale à d₅₀ ≤ 500 nm à 10 nm,
la composition totale du composant monomère liquide (A) s'élevant à 100 % en poids, **caractérisée en ce que** le matériau prothétique est présent sous forme d'un corps moulé tridimensionnel étant convenable comme ébauche de fraisage CAO/FAO, et comprend au moins un polymère comprenant au moins un (méth)acrylate d'urethane polymérisé dans le polymère, et 0,001 à 10 % en poids des particules coeur-coque modifiées par une phase élastique, par rapport à la composition totale, le corps moulé ayant une résistance à la rupture de ≥ 1.9 Mpa · m^{1/2} et une énergie totale à la rupture de ≥ 900 J/m².

2. Ébauche de fraisage selon la revendication 1, **caractérisée en ce que** les particules coeur-coque comprennent des agrégats des particules primaires, les agrégats ayant une dimension particulaire moyenne d'inférieure ou égale à (d₅₀) 400 µm (micromètres).

3. Ébauche de fraisage selon l'une des revendications 1 ou 2, comprenant iii) 0,001 à 20 % en poids au moins un (méth)acrylate d'urethane polymérisé dans le polymère, en particulier un diméthacrylate d'urethane, par rapport à la composition totale.

4. Ébauche de fraisage selon l'une des revendications 1 à 3, **caractérisée en ce que** le matériau prothétique non pigmenté a une transparence de supérieure ou égale à 90 % (mesurée par rapport aux plaques ayant une hauteur de 3 mm).

5. Ébauche de fraisage selon l'une des revendications 1 à 4, **caractérisée** en comprenant supérieure ou égale à 55 % en poids PMMA, par rapport à la composition totale.

6. Procédé pour produire un matériau prothétique polymérique sous forme d'un corps moulé, en particulier sous forme d'un corps de fraisage, dans lequel un matériau prothétique polymérisable comprenant les composants (A) et/ou (B),
- le composant
A) étant au moins composant monomère liquide, et
B) étant au moins un composant pulvérulent, et
le matériau prothétique polymérisable comprenant dans le composant (A) supérieur à 85 % en poids au moins un composant monomère mono-fonctionnel à fonction méthacrylate,
0,001 à 20 % en poids (méth)acrylate d'urethane, et
0,001 à 10 % en poids des particules coeur-coque modifiées par une phase élastique avec la phase élastique au tant qu'un cœur dans une coque extérieure solide, les particules coeur-coque ayant des particules primaires ayant une dimension particulaire moyenne d'inférieure ou égale à d₅₀ ≤ 500 nm à 10 nm,
la composition totale du composant monomère liquide (A) s'élevant à 100 % en poids,
et les composants (A) et (B)
-
(a) sont mélangés,
(b) sont transférés dans un moule tridimensionnel,
(c) et le matériau prothétique polymérisable est polymérisé ensuite.

7. Procédé selon la revendication 6, **caractérisé en ce que** le
A) composant monomère et B) composant pulvérulent sont mélangés selon un rapport en poids de 1 : 50 à 50 : 1, en particulier selon un rapport en poids de 8 à 11 composant pulvérulent à 5 à 8 composant monomère.

8. Procédé selon la revendication 6 ou 7, **caractérisé en ce que**
A) le composant monomère liquide comprend au moins un monomère ou un mélange de monomères du a) méthacrylate de méthyle, méthacrylate d'éthyle, méthacrylate de propyle, méthacrylate de butyle, méthacrylate de n-hexyle, méthacrylate de 2-phenoxyéthyle, méthacrylate d'isobornyle, méthacrylate d'isodécyle, monométhacrylate de polypropylène glycol, méthacrylate de tétrahydrofuryle, monométhacrylate de polypropylène glycol, acrylate de méthyle, acrylate d'éthyle, acrylate de propyle, acrylate de butyle, acrylate de n-hexyle, acrylate de 2-phenoxyéthyle, acrylate d'isobornyle, acrylate d'isodécyle, monoacrylate de polypropylène glycol, acrylate de tétrahydrofuryle, monoacrylate de polypropylène glycol, acrylate d'hydroxyéthyle, acrylate d'hydroxypropyle, méthacrylate d'hydroxyéthyle, méthacrylate d'hydroxypropyle, (méth)acrylates de benzyle, furfuryle ou phényle, une mélange contenant au moins un desdites (méth)acrylates et/ou des copolymères comprenant une ou au moins deux des monomères susmentionnés, et/ou b) des agents de réticulation double ou multiple comprenant le diméthacrylate de 1,4-butanediol (1,4-BDMA) ou le tétraacrylate de pentaérythritol, le monomère bis-GMA (méthacrylate de bisphénol-A-glycidyl), le diméthacrylate de triéthylène glycol (TEGDMA) et diméthacrylate de diéthylène glycol (DEGDMA), le di(méth)acrylate de tétraéthylène glycol, le di(méth)acrylate de décanediol, le di(méth)acrylate de dodécanediol, le di(méth)acrylate de décanediol de hexyle, le tri(méth)acrylate de triméthylolpropane, le tétra(méth)acrylate de pentaérythritol, ainsi que le di(méth)acrylate de butanediol, le di(méth)acrylate de éthylène glycol, le di(méth)acrylate de polyéthylène glycol, le di(méth)acrylate de bisphénol-A éthoxylé/propoxylé, une mélange contenant au moins une desdits (méth)acrylates et/ou des copolymères comprenant une ou au moins deux des monomères susmentionnés, et/ou
B) le composant pulvérulent comprend des particules polymériques comprenant des polymères sous forme de poudre de polymère comprenant des poly(méth)acrylates d'alkyle, facultativement étant réticulés et étant présents en tant qu'un homopolymère ou copolymère, le polymère étant basé sur au moins un monomère comprenant un group (méth)acrylate sélectionné parmi du méthacrylate de méthyle, méthacrylate d'éthyle, méthacrylate de propyle, méthacrylate de butyle, méthacrylate de n-hexyle, méthacrylate de 2-phenoxyéthyle, méthacrylate d'isobornyle, méthacrylate d'isodécyle, monométhacrylate de polypropylène glycol, méthacrylate de tétrahydrofuryle, acrylate de méthyle, acrylate d'éthyle, acrylate de propyle, acrylate de butyle, acrylate de n-hexyle, acrylate de 2-phenoxyéthyle, acrylate d'isobornyle, acrylate d'isodécyle, monoacrylate de polypropylène glycol, acrylate de tétrahydrofuryle, acrylate d'hydroxyéthyle, acrylate d'hydroxypropyle, méthacrylate d'hydroxyéthyle, méthacrylate d'hydroxypropyle, une mélange contenant au moins un desdits (méth)acrylates et/ou des copolymères comprenant une ou au moins deux des monomères susmentionnés, des particules de polyamide, des fibres de polyamide, particulièrement préféré est le polyméthacrylate de méthyle (PMMA).

9. Matériau prothétique polymérisé être obtenues selon un procédé selon l'une des revendications 6 à 8, **caractérisé en ce que** le matériau prothétique est présent sous forme d'un corps de fraisage tridimensionnel, en particulier sous forme d'une ébauche de fraisage.

10. Matériau prothétique polymérisé selon la revendication 9, caractérisé en ayant une résistance à la rupture de ≥ 1.9 Mpa · m^{1/2} et une énergie totale à la rupture de ≥ 900 J/m².

11. Utilisant d'une ébauche de fraisage selon l'une des revendications 1 à 5, pour produire des composantes, en particulière des composantes dentaires ou orthopédiques, comprenant des panneaux de base prothétique, des attelles occlusales, des couronnes, des bridges, des dents artificielles, des habillages, des inlays, des onlays, des appareils orthodontiques, des appareils orthodontiques actifs ou passifs, des appareils Crozat, des activateurs modifiés, des implants, des superstructures, des barres dentaires, des piliers, des dispositifs de fixation dentaires, des vis dentaires, des guides chirurgicaux pour l'implantologie, des prothèses télescopiques et des couronnes télescopiques, des facettes, des gouttières occlusales, des prothèses articulaires artificielles, des appareils dentaires, des plaques double de propulsion, des parties d'implant, des appareils dentaires invisibles, des supports, des instruments orthodontiques et/ou des appareils multibandes, ou d'au moins des parties des composantes susmentionnées, ou dans le domaine vétérinaire, en particulier pour des composantes de réparation des sabots.
